(19) Europäisches Patentamt
European Patent Office
Office européen des brevets

(11) EP 2 489 345 B1

(12) EUROPÄISCHE PATENTSCHRIFT

(45) Veröffentlichungstag und Bekanntmachung des Hinweises auf die Patenterteilung:
**13.04.2016 Patentblatt 2016/15**

(51) Int Cl.:
***A61K 8/81*** *(2006.01)* ***A61Q 5/06*** *(2006.01)*

(21) Anmeldenummer: **12157224.2**

(22) Anmeldetag: **16.06.2008**

(54) **Stylingmittel mit hohem Haltegrad bei Feuchtigkeit III**

Styling agents giving a high degree of hold III

Produits de coiffage apportant une tenue forte en atmosphère humide III

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **09.11.2007 DE 102007053953**

(43) Veröffentlichungstag der Anmeldung:
**22.08.2012 Patentblatt 2012/34**

(62) Dokumentnummer(n) der früheren Anmeldung(en) nach Art. 76 EPÜ:
**08761055.6 / 2 205 208**

(73) Patentinhaber: **Henkel AG & Co. KGaA**
**40589 Düsseldorf (DE)**

(72) Erfinder:
- **Knappe, Thorsten**
  **22869 Schenefeld (DE)**
- **Scheffler, Rene**
  **25479 Ellerau (DE)**

(56) Entgegenhaltungen:
**WO-A2-02/083073 DE-A1- 2 103 898**
**DE-A1- 2 822 358 DE-A1- 19 738 866**
**FR-A1- 2 786 391**

- **ROHM AND HAAS COMPANY: "Uses of Ethylenecarboxamide / Acrylamidomethylpropanesulfonate / Methacrylates polymer in personal care applications: Acudyne SCP styling conditioning polymer", RESEARCH DISCLOSURE, MASON PUBLICATIONS, HAMPSHIRE, GB, Bd. 478, Nr. 2, 1. Februar 2004 (2004-02-01), XP007133356, ISSN: 0374-4353**
- **"Acudyne Hair Fixative Resins", INTERNET CITATION, 18. Oktober 2006 (2006-10-18), Seiten 1-2, XP007908245, Gefunden im Internet: URL:http://web.archive.org/web/20061018193137/www.rhpersonalcare.com/acudyne_scp [gefunden am 2009-04-17]**
- **"Hydroxyethyl Urea in Personal Care and Hair Spray Formulations with acrylates copolymer to Improve Aesthetic and Spray Properties", IP.COM JOURNAL, IP.COM INC., WEST HENRIETTA, NY, US, 15. August 2006 (2006-08-15), XP013115494, ISSN: 1533-0001**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft Mittel zur temporären Verformung keratinischer Fasern, enthaltend eine spezielle Kombination von Polymeren, die Verwendung dieser Mittel zur temporären Verformung keratinischer Fasern und Aerosolhaarschäume auf Basis dieser Mittel. Unter keratinischen Fasern werden prinzipiell alle tierischen Haare, z.B. Wolle, Rosshaar, Angorahaar, Pelze, Federn und daraus gefertigte Produkte oder Textilien verstanden. Vorzugsweise handelt es sich bei den keratinischen Fasern jedoch um menschliche Haare.

Eine ansprechend aussehende Frisur wird heute allgemein als unverzichtbarer Teil eines gepflegten Äußeren angesehen. Dabei gelten aufgrund von aktuellen Modeströmungen immer wieder Frisuren als chic, die sich bei vielen Haartypen nur unter Verwendung festigender Wirkstoffe aufbauen bzw. für einen längeren Zeitraum bis hin zu mehreren Tagen aufrechterhalten lassen. Daher spielen Haarbehandlungsmittel, die einer permanenten oder temporären Formgebung der Haare dienen, eine wichtige Rolle. Temporäre Formgebungen, die einen guten Halt ergeben sollen, ohne das gesunde Aussehen der Haare, wie zum Beispiel deren Glanz, zu beeinträchtigen, können beispielsweise durch Haarsprays, Haarwachse, Haargele, Haarschäume, Fönwellen etc. erzielt werden.

Entsprechende Mittel zur temporären Formgebung enthalten als formgebende Komponente üblicherweise synthetische Polymere. Zubereitungen, die ein gelöstes oder dispergiertes Polymer enthalten, können mittels Treibgasen oder durch einen Pumpmechanismus auf das Haar aufgebracht werden. Insbesondere Haargele und Haarwachse werden allerdings in der Regel nicht direkt auf das Haar appliziert, sondern mittels eines Kamms oder der Hände im Haar verteilt.

Die wichtigste Eigenschaft eines Mittels zur temporären Verformung keratinischer Fasern, im Folgenden auch Stylingmittel genannt, besteht darin, den behandelten Fasern in der erzeugten Form einen möglichst starken Halt zu geben. Handelt es sich bei den keratinischen Fasern um menschliche Haare, spricht man auch von starkem Frisurenhalt oder vom hohen Haltegrad des Stylingmittels. Der Frisurenhalt wird im Wesentlichen durch die Art und Menge des eingesetzten synthetischen Polymers bestimmt, wobei jedoch auch ein Einfluss der weiteren Bestandteile des Stylingmittels gegeben sein kann.

Neben einem hohen Haltegrad müssen Stylingmittel eine ganze Reihe weiterer Anforderungen erfüllen. Diese können grob in Eigenschaften am Haar, Eigenschaften der jeweiligen Formulierung, z. B. Eigenschaften des Schaums, des Gels oder des versprühten Aerosols, und Eigenschaften, die die Handhabung des Stylingmittels betreffen, unterteilt werden, wobei den Eigenschaften am Haar besondere Wichtigkeit zukommt. Zu nennen sind insbesondere Feuchtebeständigkeit, niedrige Klebrigkeit und ein ausgewogener Konditioniereffekt. Weiterhin soll ein Stylingmittel möglichst für alle Haartypen universell einsetzbar sein.

Um den unterschiedlichen Anforderungen gerecht zu werden, wurde bereits eine Vielzahl von synthetischen Polymeren entwickelt, die in Stylingmitteln zur Anwendung kommen. Die Polymere lassen sich in kationische, anionische, nichtionische und amphotere filmbildende und/oder festigende Polymere unterteilen. Idealerweis ergeben die Polymere bei der Anwendung auf dem Haar einen Polymerfilm, der einerseits der Frisur einen starken Halt verleiht, andererseits aber hinreichend flexibel ist, um bei Beanspruchung nicht zu brechen. Ist der Polymerfilm zu brüchig, kommt es zur Bildung so genannter Filmplaken, das heißt Rückständen, die sich bei der Bewegung des Haares ablösen und den Eindruck vermitteln, der Anwender des entsprechenden Stylingmittels hätte Schuppen.

Stylingmittel zu entwickeln, die alle gewünschten Eigenschaften in Kombination aufweisen, bereitet nach wie vor Schwierigkeiten. Insbesondere gilt dies für Stylingmittel, die einen besonders starken Halt aufweisen sollen.

Aufgabe der vorliegenden Erfindung war es daher, ein Mittel zur temporären Verformung keratinischer Fasern zur Verfügung zu stellen, das sich durch einen sehr hohen Haltegrad auszeichnet ohne daß dabei auf Flexibilität und gute Feuchtebeständigkeit verzichtet werden müsste.

Es wurde nunmehr überraschenderweise gefunden, daß dies durch eine Kombination spezieller Polymere erreicht werden kann.

Ein erster Gegenstand der vorliegenden Erfindung ist daher ein kosmetisches Mittel, enthaltend in einem kosmetisch akzeptablen Träger bezogen auf das Gewicht des anwendungsbereiten Mittels

a) 0,25 bis 5,0 Gew.-% mindestens eines Copolymer A, gebildet aus

- mindestens einem Monomer A1 ausgewählt aus Acrylsäure und/oder Methacrylsäure, und
- mindestens einem Monomer A2 ausgewählt aus Acrylamid und/oder Methacrylamid und
- mindestens einem Monomer A3 ausgewählt aus Acryloyldimethyltaurat

b) und 0,25 bis 5,0 Gew.-% mindestens eines von Copolymer A unterschiedliches kationisches Polymer B, ausgewählt aus

b2) Copolymeren von Vinylpyrrolidon mit Dimethylaminoethylmethacrylat,

dadurch gekennzeichnet, dass das Gewichtsverhältnis von Polymer(en) A zu Polymer(en) B 4:1 bis 1:4 beträgt.

Filmbildende und/oder festigende Copolymere A sind bekannt. Gleiches gilt für kationische Polymere B und ihre Verwendung als filmbildende und/oder festigende Polymere. Es hat sich nun überraschenderweise gezeigt, dass eine entsprechende Kombination beider Polymertypen selbstverdickende Eigenschaften besitzt, wobei die ausgezeichneten filmbildenden und/oder festigenden Eigenschaften der einzelnen Polymere noch erhöht werden. Stylingmittel, enthaltend eine Kombination dieser Polymere, zeichnen sich durch eine synergistische Erhöhung des Haltegrads und gute Feuchtebeständigkeit des erzielten Halts aus, ohne dabei in ihrer Auswaschbarkeit beeinträchtigt zu werden.

Als ersten zwingenden Bestandteil enthalten die erfindungsgemäßen kosmetischen Mittel mindestens ein Copolymer A. Dieses Copolymer beinhaltet mindestens ein Monomer A1 ausgewählt aus Acrylsäure und/oder Methacrylsäure, und mindestens ein Monomer A2 ausgewählt aus Acrylamid und/oder Methacrylamid und mindestens ein Monomer A3 ausgewählt aus N-substituierten Acrylamiden und/oder Methacrylamiden und kann darüber hinaus weitere Struktureinheiten aufweisen, die durch das Hinzufügen entsprechender Monomere bei der Polymerisation einpolymerisiert werden.

Besonders bevorzugte erfindungsgemäße Mittel sind dadurch gekennzeichnet, daß sie als Copolymer A ein Copolymer A1 enthalten, welches als Monomer A1 Acrylsäure umfaßt.

Ein bevorzugtes Monomer ist das Acrylamid. Demnach sind bevorzugte erfindungsgemäße Mittel dadurch gekennzeichnet, daß sie als Copolymer A ein Copolymer A1 enthalten, welches als Monomer A2 Acrylamid umfaßt.

Die N-Substitution an den N-substituierten Acrylamiden kann durch einfache Alkalgruppen (vorzugsweise Methyl-, Ethyl-, n-Propyl, Isoporpyl) erfolgen, besonders bevorzugt sind jedoch substituierte Alkylgruppen, die anionische Funktionalitäten tragen. Ganz besonders bevorzugt sind Sulfonatgruppenhaltige Substituenten.

Ein erfindungsgemäßes Mittel ist dadurch gekennzeichnet, daß es als Copolymer A ein Copolymer A1 enthält, welches als Monomer A3 Acryloyldimethyltaurat umfaßt.

Diese lassen sich durch die allgemeine Formel

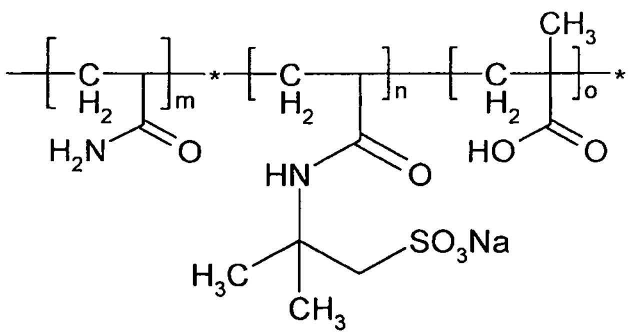

beschreiben, wobei die Indices m, n und o je nach Molmasse des Polymers variieren und nicht bedeuten sollen, daß es sich um Blockcopolymere handelt. Vielmehr können Struktureinheiten im Molekül statistisch verteilt vorliegen.

Besonders bevorzugte erfindungsgemäße Mittel sind dadurch gekennzeichnet, daß das Copolymer A1 eine Molmasse von 50 bis 500 kDa, vorzugsweise von 100 bis 450 kDa, weiter bevorzugt von 150 bis 400 kDa und insbesondere von 200 bis 300 kDa aufweist.

Vorzugsweise werden die Copolymere A innerhalb bestimmter Mengenbereiche eingesetzt. Hier sind erfindungsgemäße Mittel bevorzugt, bei denen die Gesamtmenge an Copolymeren A, bezogen auf das Gewicht des anwendungsbereiten Mittels, 0,25 bis 3 Gew.-% beträgt. Copolymere von Acrylamid mit Methacrylsäure und Acryloyldimethyltaurat sind beispielsweise unter dem Handelsnamen Acudyne® SCP (Rohm & Haas) erhältlich.

Die erfindungsgemäßen Mittel enthalten mindestens ein weiteres Polymer B aus der Gruppe der kationischen Polymere, d.h. der Polymere, die mindestens eine Monomereinheit mit einer positiv geladenen Gruppe enthalten. Dieses kationische Polymer ist ausgewählt aus b2) Copolymeren von Vinylpyrrolidon mit Dimethylaminoethylmethacrylat.

Die erfindungsgemäßen Mittel enthalten auch Polymere b2 aus der Gruppe der Copolymeren von Vinylpyrrolidon mit Dimethylaminoethylmethacrylat. Diese lassen sich durch die allgemeine Formel

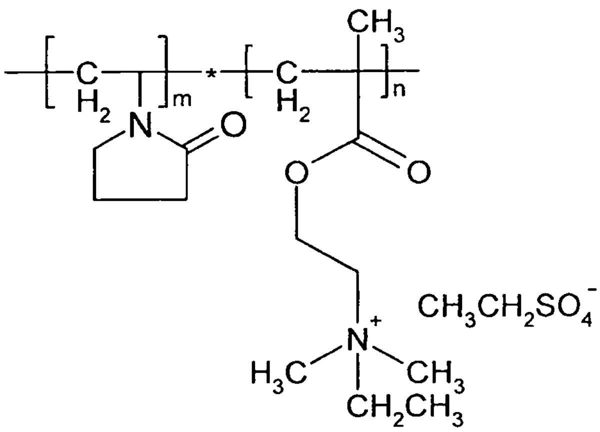

beschreiben, wobei die Indices m und n je nach Molmasse des Polymers variieren und nicht bedeuten sollen, daß es sich um Blockcopolymere handelt. Vielmehr können Struktureinheiten im Molekül statistisch verteilt vorliegen.

Besonders bevorzugte erfindungsgemäße Mittel sind dadurch gekennzeichnet, daß sie als kationisches Polymer b2 Copolymere von Vinylpyrrolidon mit Dimethylaminoethylmethacrylat enthält, die 40 bis 95 Mol.-%, vorzugsweise 42,5 bis 90 Mol.-%, weiter bevorzugt 45 bis 85 Mol.-% und insbesondere 50 bis 80 Mol.-% Vinylpyrrolidon enthalten

Besonders bevorzugte erfindungsgemäße Mittel sind weiter dadurch gekennzeichnet, daß die Copolymere b2 Molmassen von 100 bis 2500 kDa, vorzugsweise von 250 bis 2000 kDa, weiter bevorzugt von 500 bis 1750 kDa und insbesondere von 800 bis 1500 kDa, aufweisen. Vorzugsweise werden die Copolymere b2 innerhalb bestimmter Mengenbereiche eingesetzt. Hier sind erfindungsgemäße Mittel bevorzugt, die, bezogen auf das Gewicht des anwendungsbereiten Mittels, 0,05 bis 5 Gew.-%, vorzugsweise 0,1 bis 4 Gew.-% und insbesondere 0,25 bis 3 Gew.-% Copolymer(e) b4 enthalten.

Ein ganz besonders bevorzugtes Copolymer b2 wird nach der INCI-Nomenklatur als Polyquaternium-11 bezeichnet. Ein solches Polymer ist beispielsweise unter der Handelsbezeichnung Gafquat® 755 N (ISP) erhältlich.

Unabhängig davon, welche der Polymerkombinationen gewählt wird, sind erfindungsgemäße Mittel dadurch gekennzeichnet, dass das Gewichtsverhältnis von Polymer(en) A zu Polymer(en) B 4:1 bis 1:4 beträgt.

Unabhängig von Art und Gewichtsverhältnis der Polymeren zueinander sind darüber hinaus erfindungsgemäße Mittel bevorzugt, bei denen der Gesamt-Polymergehalt der Mittel 1 bis 15 Gew.-%, vorzugsweise 2,5 bis 12,5 Gew.-%, weiter bevorzugt 4 bis 10 Gew.-% und insbesondere 5 bis 8 Gew.-% beträgt.

Die erfindungsgemäßen Mittel enthalten die Polymere in einem kosmetisch akzeptablen Träger.

Bevorzugte kosmetisch akzeptable Träger sind wässrige, alkoholische oder wässrigalkoholische Medien mit vorzugsweise mindestens 10 Gew.-% Wasser, bezogen auf das gesamte Mittel. Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein. Als zusätzliche Co-Solventien können organische Lösungsmittel oder ein Gemisch aus Lösungsmitteln mit einem Siedepunkt unter 400°C in einer Menge von 0,1 bis 15 Gewichtsprozent, bevorzugt von 1 bis 10 Gewichtsprozent bezogen auf das gesamte Mittel enthalten sein. Besonders geeignet als zusätzliche Co-Solventien sind unverzweigte oder verzweigte Kohlenwasserstoffe wie Pentan, Hexan, Isopentan und cyclische Kohlenwasserstoffe wie Cyclopentan und Cyclohexan. Weitere, besonders bevorzugte wasserlösliche Lösungsmittel sind Glycerin, Ethylenglykol Butylenglycol, Sorbitol und Propylenglykol in einer Menge bis 30 Gew.-% bezogen auf das gesamte Mittel.

Die Mittel weisen bevorzugt einen pH-Wert von 2 bis 11 auf. Besonders bevorzugt ist der pH-Bereich zwischen 2 und 8. Die Angaben zum pH-Wert beziehen sich dabei im Sinne dieser Schrift auf den pH-Wert bei 25°C, sofern nichts anderes vermerkt ist.

Die erfindungsgemäßen Mittel können weiterhin die Hilfs- und Zusatzstoffe enthalten, die üblicherweise den jeweiligen kosmetischen Mitteln zugesetzt werden.

Als geeignete Hilfs- und Zusatzstoffe sind insbesondere Pflegestoffe zu nennen. Diese finden sowohl bei Haut- als auch Haarbehandlungsmitteln Anwendung und können bei geeigneter Wahl des Pflegestoffs beispielsweise in Cremes, Shampoos, Haarspülungen, Haarkuren, Gele, Pump- und Aerosolsprays und Schaumprodukte eingearbeitet werden.

Als Pflegestoff kann beispielsweise ein Silikonöl und/oder ein Silikongum eingesetzt werden. In einer besonderen Ausführungsform der Erfindung enthalten die Mittel mindestens ein Silikonöl und/oder ein Silikongum.

Erfindungsgemäß geeignete Silikonöle oder Silikongums sind insbesondere Dialkyl- und Alkylarylsiloxane, wie beispielsweise Dimethylpolysiloxan und Methylphenylpolysiloxan, sowie deren alkoxylierte, quaternierte oder auch anionische Derivate. Bevorzugt sind cyclische und lineare Polydialkylsiloxane, deren alkoxylierte und/oder aminierte Derivate, Dihydroxypolydimethylsiloxane und Polyphenylalkylsiloxane.

Die Mittel enthalten die Silikone bevorzugt in Mengen von 1 - 25 Gew.-%, besonders bevorzugt von 5 - 20 Gew.-% und insbesondere bevorzugt von 7 - 15 Gew.-%, bezogen auf das gesamte Mittel.

Obwohl das erfindungsgemäße Mittel als Pflegestoff vorzugsweise ein Silikonderivat enthält, ist es auch möglich, dass das Mittel statt oder neben einer Silikonkomponente mindestens einen Pflegestoff einer anderen Verbindungsklasse enthält.

Als Pflegestoff einer anderen Verbindungsklasse kann das Mittel beispielsweise mindestens ein Proteinhydrolysat und/oder eines seiner Derivate enthalten.

Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Zu letzteren sind beispielsweise Polyalanin, Polyasparagin, Polyserin etc. zu zählen. Weitere Beispiele für erfindungsgemäß einsetzbare Verbindungen sind L-Alanyl-L-prolin, Polyglycin, Glycyl-L-glutamin oder D/L-Methionin-S-Methylsulfoniumchlorid. Selbstverständlich können erfindungsgemäß auch ß-Aminosäuren und deren Derivate wie ß-Alanin, Anthranilsäure oder Hippursäure eingesetzt werden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton. Erfindungsgemäß können Proteinhydrolysate sowohl pflanzlichen als auch tierischen oder marinen oder synthetischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan® (Cognis), Promois® (Interorgana), Collapuron® (Cognis), Nutrilan® (Cognis), Gelita-Sol® (Deutsche Gelatine Fabriken Stoess & Co), Lexein® (Inolex), Sericin (Pentapharm) und Kerasol® (Croda) vertrieben.

Als Pflegestoff einer anderen Verbindungsklasse sind weiterhin kationische Tenside geeignet. Erfindungsgemäß bevorzugt sind kationische Tenside vom Typ der quartären Ammoniumverbindungen, der Esterquats und der Amidoamine. Bevorzugte quartäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z.B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis(2-Palmitoyloxyethyl)dimethylammoniumchlorid, sowie Dehyquart® F-75, Dehyquart® C-4046, Dehyquart® L80 und Dehyquart® AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyldimethylamin dar.

Die kationischen Tenside sind in den erfindungsgemäßen Mitteln bevorzugt in Mengen von 0,05 bis 10 Gew.-%, bezogen auf die gesamte Anwendungszubereitung, enthalten. Mengen von 0,1 bis 5 Gew.-% sind besonders bevorzugt.

Als Pflegestoff eignen sich ebenfalls pflegende Polymere. Es sei an dieser Stelle darauf hingewiesen, dass einige pflegende Polymere auch filmbildende und/oder festigende Eigenschaften aufweisen, und daher auch bei der Aufzählung geeigneter filmbildender und/oder festigender Polymere genannt sein können.

Eine erste Gruppe der pflegenden Polymere sind die kationischen Polymere. Unter kationischen Polymeren sind Polymere zu verstehen, welche in der Haupt- und/oder Seitenkette eine Gruppe aufweisen, welche "temporär" oder "permanent" kationisch sein kann. als. "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine $C_{1-4}$-Kohlenwasserstoffgruppe an eine aus Acrylsäure, Methacrylsäure oder deren Derivaten aufgebaute Polymerhauptkette gebunden sind, haben sich als besonders geeignet erwiesen.

Homopolymere der allgemeinen Formel (G1-I),

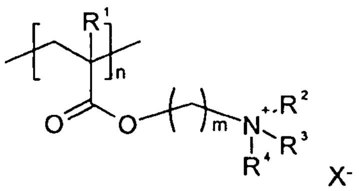

(G1-I)

in der $R^1$= -H oder $-CH_3$ ist, $R^2$, $R^3$ und $R^4$ unabhängig voneinander ausgewählt sind aus $C_{1-4}$-Alkyl-, -Alkenyl- oder -Hydroxyalkylgruppen, m = 1, 2, 3 oder 4, n eine natürliche Zahl und X- ein physiologisch verträgliches organisches oder anorganisches Anion ist, sowie Copolymere, bestehend im wesentlichen aus den in Formel (G1-I) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:

$R^1$ steht für eine Methylgruppe
$R^2$, $R^3$ und $R^4$ stehen für Methylgruppen
m hat den Wert 2.

Als physiologisch verträgliche Gegenionen X- kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwässrigen Polymerdispersion, die einen Polymeranteil nicht unter 30 Gew.-% aufweisen sollte, eingesetzt. Solche Polymerdispersionen sind unter den Bezeichnungen Salcare® SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare® SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich. Copolymere mit Monomereinheiten gemäß Formel (G1-I) enthalten als nichtionogene Monomereinheiten bevorzugt Acrylamid, Methacrylamid, Acrylsäure-$C_{1-4}$-alkylester und Methacrylsäure-$C_{1-4}$-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare® SC 92 erhältlich.

Weitere bevorzugte kationische Polymere sind beispielsweise

- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR®400 sind bevorzugte quaternierte Cellulose-Derivate,
- kationische Alkylpolyglycoside gemäß der DE-PS 44 13 686,
- kationiserter Honig, beispielsweise das Handelsprodukt Honeyquat® 50,
- kationische Guar-Derivate, wie insbesondere die unter den Handelsnamen Cosmedia®Guar und Jaguar® vertriebenen Produkte,
- Polysiloxane mit quartären Gruppen, wie beispielsweise die im Handel erhältlichen Produkte Q2-7224 (Hersteller: Dow Corning; ein stabilisiertes Trimethylsilylamodimethicon), Dow Corning® 929 Emulsion (enthaltend ein hydroxylamino-modifiziertes Silikon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt), diquaternäre Polydimethylsiloxane, Quaternium-80),
- polymere Dimethyldiallylammoniumsalze und deren Copolymere mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammoniumchlorid-Acrylamid-Copolymer) im Handel erhältlichen Produkte sind Beispiele für solche

kationischen Polymere,

- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat®734 und Gafquat®755 im Handel erhältlich,
- Vinylpyrrolidon-Vinylimidazoliummethochlorid-Copolymere, wie sie unter den Bezeichnungen Luviquat® FC 370, FC 550, FC 905 und HM 552 angeboten werden,
- quaternierter Polyvinylalkohol,
- sowie die unter den Bezeichnungen Polyquaternium 2, Polyquaternium 17, Polyquaternium 18 und Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft® LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix® VC 713 (Hersteller: ISP), Gafquat®ASCP 1011, Gafquat®HS 110, Luviquat®8155 und Luviquat® MS 370 erhältlich sind.

Weitere erfindungsgemäß einsetzbare kationische Polymere sind die so genannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen® CMF, Hydagen® HCMF, Kytamer® PC und Chitolam® NB/101 im Handel frei verfügbar sind.

Erfindungsgemäß bevorzugt eingesetzte kationische Polymere sind kationische Cellulose-Derivate und Chitosan und dessen Derivate, insbesondere die Handelsprodukte Polymer®JR 400, Hydagen® HCMF und Kytamer® PC, kationische Guar-Derivate, kationische Honig-Derivate, insbesondere das Handelsprodukt Honeyquat® 50, kationische Alkylpolyglycodside gemäß der DE-PS 44 13 686 und Polymere vom Typ Polyquaternium-37.

Weiterhin sind kationisierte Proteinhydrolysate zu den kationischen Polymeren zu zählen, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder biotechnologisch gewonnenen Proteinhydrolysaten, stammen kann. Die den erfindungsgemäßen kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder eine Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quartären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die erfindungsgemäßen kationischen Proteinhydrolysate und -derivate seien die unter den INCI - Bezeichnungen im "International Cosmetic Ingredient Dictionary and Handbook", (seventh edition 1997, The Cosmetic, Toiletry, and Fragrance Association 1101 17th Street, N.W., Suite 300, Washington, DC 20036-4702) genannten und im Handel erhältlichen Produkte genannt: Cocodimonium Hydroxypropyl Hydrolyzed Collagen, Cocodimonium Hydroxypropyl Hydrolyzed Casein, Cocodimonium Hydroxypropyl Hydrolyzed Collagen, Cocodimonium Hydroxypropyl Hydrolyzed Hair Keratin, Cocodimonium Hydroxypropyl Hydrolyzed Keratin, Cocodimonium Hydroxypropyl Hydrolyzed Rice Protein,. Cocodimonium Hydroxypropyl Hydrolyzed Soy Protein, Cocodimonium Hydroxypropyl Hydrolyzed Wheat Protein, Hydroxypropyl Arginine Lauryl/Myristyl Ether HCl, Hydroxypropyltrimonium Gelatin, Hydroxypropyltrimonium Hydrolyzed Casein, Hydroxypropyltrimonium Hydrolyzed Collagen, Hydroxypropyltrimonium Hydrolyzed Conchiolin Protein, Hydroxypropyltrimonium Hydrolyzed Keratin, Hydroxypropyltrimonium Hydrolyzed Rice Bran Protein, Hydroxypropyltrimonium Hydrolyzed Soy Protein, Hydroxypropyl Hydrolyzed Vegetable Protein, Hydroxypropyltrimonium Hydrolyzed Wheat Protein, Hydroxypropyltrimonium Hydrolyzed Wheat Protein/Siloxysilicate, Laurdimonium Hydroxypropyl Hydrolyzed Soy Protein, Laurdimonium Hydroxypropyl Hydrolyzed Wheat Protein, Laurdimonium Hydroxypropyl Hydrolyzed Wheat Protein/Siloxysilicate, Lauryldimonium Hydroxypropyl Hydrolyzed Casein, Lauryldimonium Hydroxypropyl Hydrolyzed Collagen, Lauryldimonium Hydroxypropyl Hydrolyzed Keratin, Lauryldimonium Hydroxypropyl Hydrolyzed Soy Protein, Steardimonium Hydroxypropyl Hydrolyzed Casein, Steardimonium Hydroxypropyl Hydrolyzed Collagen, Steardimonium Hydroxypropyl Hydrolyzed Keratin, Steardimonium Hydroxypropyl Hydrolyzed Rice Protein, Steardimonium Hydroxypropyl Hydrolyzed Soy Protein, Steardimonium Hydroxypropyl Hydrolyzed Vegetable Protein, Steardimonium Hydroxypropyl Hydrolyzed Wheat Protein, Steartrimonium Hydroxyethyl Hydrolyzed Collagen, Quaternium-76 Hydrolyzed Collagen, Quaternium-79 Hydrolyzed Collagen, Quaternium-79 Hydrolyzed Keratin, Quaternium-79 Hydrolyzed Milk Protein, Quaternium-79 Hydrolyzed Soy Protein, Quaternium-79 Hydrolyzed Wheat Protein.

Ganz besonders bevorzugt sind die kationischen Proteinhydrolysate und -derivate auf pflanzlicher Basis.

Bevorzugt eingesetzte amphotere Polymere sind solche Polymerisate, die sich im wesentlichen zusammensetzen aus

(a) Monomeren mit quartären Ammoniumgruppen der allgemeinen Formel (II),

$$R^1\text{-CH=CR}^2\text{-CO-Z-}(C_nH_{2n})\text{-N}^{(+)}R^3R^4R^5\ A^{(-)} \qquad \text{(II)}$$

in der $R^1$ und $R^2$ unabhängig voneinander stehen für Wasserstoff oder eine Methylgruppe und $R^3$, $R^4$ und $R^5$ jeweils unabhängig voneinander für eine Alkylgruppe mit 1 bis 4 Kohlenstoffatomen, Z eine NH-Gruppe oder ein Sauerstoffatom, n eine ganze Zahl von 2 bis 5 und $A^{(-)}$ das Anion einer organischen oder anorganischen Säure ist, und
(b) monomeren Carbonsäuren der allgemeinen Formel (III),

$$R^6\text{-CH=CR}^7\text{-COOH} \qquad \text{(III)}$$

in denen $R^6$ und $R^7$ unabhängig voneinander für Wasserstoff oder eine Methylgruppe stehen.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Ganz besonders bevorzugt sind solche Polymerisate, bei denen Monomere des Typs (a) eingesetzt werden, bei denen $R^3$, $R^4$ und $R^5$ Methylgruppen sind, Z eine NH-Gruppe und $A^{(-)}$ ein Halogenid-, Methoxysulfat- oder Ethoxysulfat-Ion ist; Acrylamidopropyltrimethylammoniumchlorid ist ein besonders bevorzugtes Monomeres (a). Als Monomeres (b) für die genannten Polymerisate wi rd bevorzugt Acrylsäure verwendet.

Die erfindungsgemäßen Mittel enthalten die pflegenden, kationischen und/oder amphoteren Polymere in bevorzugter Weise in einer Menge von 0,01 bis 5 Gew.-%, insbesondere in einer Menge von 0,1 bis 2 Gew.-%, jeweils bezogen auf die gesamte Anwendungszubereitung.

Als Pflegestoff kann das erfindungsgemäße Mittel weiterhin mindestens ein Vitamin, ein Provitamin, eine Vitaminvorstufe und/oder eines derer Derivate enthalten. Dabei sind erfindungsgemäß solche Vitamine, Provitamine und Vitaminvorstufen bevorzugt, die üblicherweise den Gruppen A, B, C, E, F und H zugeordnet werden. Panthenol, Pantolacton, Pyridoxin und seine Derivate sowie Nicotinsäureamid und Biotin sind besonders bevorzugt. Ganz besonders bevorzugt wird als Pflegestoff D-Panthenol, gegebenenfalls in Kombination mit mindestens einem der oben genannten Silikonderivate eingesetzt.

Als Pflegestoff können die erfindungsgemäßen Mittel weiterhin mindestens einen Pflanzenextrakt enthalten.

Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen.

Erfindungsgemäß sind vor allem die Extrakte aus Grünem Tee, Eichenrinde, Brennnessel, Hamamelis, Hopfen, Henna, Kamille, Klettenwurzel, Schachtelhalm, Weißdorn, Lindenblüten, Mandel, Aloe Vera, Fichtennadel, Rosskastanie, Sandelholz, Wacholder, Kokosnuss, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel bevorzugt.

Besonders bevorzugt sind die Extrakte aus Grünem Tee, Eichenrinde, Brennnessel, Hamamelis, Hopfen, Kamille, Klettenwurzel, Schachtelhalm, Lindenblüten, Mandel, Aloe Vera, Kokosnuss, Mango, Aprikose, Limone, Weizen, Kiwi, Melone, Orange, Grapefruit, Salbei, Rosmarin, Birke, Wiesenschaumkraut, Quendel, Schafgarbe, Hauhechel, Meristem, Ginseng und Ingwerwurzel. Ganz besonders geeignet sind die Extrakte aus Grünem Tee, Mandel, Aloe Vera, Kokosnuss, Mango, Aprikose, Limone, Weizen, Kiwi und Melone.

Auch Mono- bzw. Oligosaccharide können als Pflegestoff in den erfindungsgemäßen Mitteln eingesetzt werden. Es können sowohl Monosaccharide als auch Oligosaccharide, wie beispielweise Rohrzucker, Milchzucker und Raffinose, eingesetzt werden. Die Verwendung von Monosacchariden ist erfindungsgemäß bevorzugt. Unter den Monosacchariden sind wiederum solche Verbindungen bevorzugt, die 5 oder 6 Kohlenstoffatome enthalten. Geeignete Pentosen und Hexosen sind beispielsweise Ribose, Arabinose, Xylose, Lyxose, Allose, Altrose, Glucose, Mannose, Gulose, Idose, Galactose, Talose, Fucose und Fructose. Arabinose, Glucose, Galactose und Fructose sind bevorzugt eingesetzte Kohlenhydrate; Ganz besonders bevorzugt eingesetzt wi rd Glucose, die sowohl in der D-(+)- oder L-(-)- Konfiguration oder als Racemat geeignet ist. Weiterhin können auch Derivate dieser Pentosen und Hexosen, wie die entsprechenden On- und Uronsäuren (Zuckersäuren), Zuckeralkohole und Glykoside, erfindungsgemäß eingesetzt werden. Bevorzugte Zuckersäuren sind die Gluconsäure, die Glucuronsäure, die Zuckersäure, die Mannozuckersäure und die Schleimsäure. Bevorzugte Zuckeralkohole sind Sorbit, Mannit und Dulcit. Bevorzugte Glykoside sind die Methylglucoside.

Die Mono- bzw. Oligosaccharide sind in den erfindungsgemäßen Mitteln bevorzugt in einer Menge von 0,1 bis 8 Gew.-%, insbesondere bevorzugt 1 bis 5 Gew.-%, bezogen auf die gesamte Anwendungszubereitung, enthalten.

Das Mittel kann weiterhin mindestens ein Lipid als Pflegestoff enthalten.

Erfindungsgemäß geeignete Lipide sind Phospholipide, beispielsweise Sojalecithin, Ei-Lecithin und Kephaline sowie die unter den INCI-Bezeichnungen Linoleamidopropyl PG-Dimonium Chloride Phosphate, Cocamidopropyl PG-Dimonium Chloride Phosphate und Stearamidopropyl PG-Dimonium Chloride Phosphate bekannten Substanzen. Diese werden beispielsweise von der Firma Mona unter den Handelsbezeichnungen Phospholipid EFA®, Phospholipid PTC® sowie Phospholipid SV® vertrieben.

Die erfindungsgemäßen Mittel enthalten die Lipide bevorzugt in Mengen von 0,01 - 10 Gew.-%, insbesondere 0,1- 5 Gew.-%, bezogen auf die gesamte Anwendungszubereitung.

Weiterhin sind als Pflegestoff Ölkörper geeignet.

Zu den natürlichen und synthetischen kosmetischen Ölkörpern sind beispielsweise zu zählen:

- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol® S) und Di-n-octylether (Cetiol® OE) können bevorzugt sein.
- Esteröle. Unter Esterölen sind zu verstehen die Ester von $C_6$ - $C_{30}$ - Fettsäuren mit $C_2$ - $C_{30}$ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkoholcaprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol® CC),
- Trifettsäureester von gesättigten und/oder ungesättigten linearen und/oder verzweigten Fettsäuren mit Glycerin,
- Fettsäurepartialglyceride, worunter Monoglyceride, Diglyceride und deren technische Gemische zu verstehen sind. Bei der Verwendung technischer Produkte können herstellungsbedingt noch geringe Mengen Triglyceride enthalten sein. Die Partialglyceride folgen vorzugsweise der Formel (D4-I),

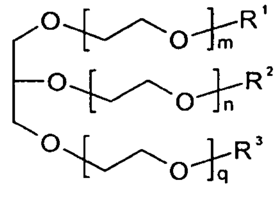

(D4-I)

in der $R^1$, $R^2$ und $R^3$ unabhängig voneinander für Wasserstoff oder für einen linearen oder verzweigten, gesättigten und/oder ungesättigten Acylrest mit 6 bis 22, vorzugsweise 12 bis 18, Kohlenstoffatomen stehen mit der Maßgabe, dass mindestens eine dieser Gruppen für einen Acylrest und mindestens eine dieser Gruppen für Wasserstoff steht. Die Summe (m+n+q) steht für 0 oder Zahlen von 1 bis 100, vorzugsweise für 0 oder 5 bis 25. Bevorzugt steht $R^1$ für einen Acylrest und $R^2$ und $R^3$ für Wasserstoff und die Summe (m+n+q) ist 0. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Ölsäuremonoglyceride eingesetzt.

Die Einsatzmenge der natürlichen und synthetischen kosmetischen Ölkörper in den erfindungsgemäßen Mitteln beträgt üblicherweise 0,1 - 30 Gew.%, bezogen auf die gesamte Anwendungszubereitung, bevorzugt 0,1 - 20 Gew.-%, und insbesondere 0,1 - 15 Gew.-%.

Das Mittel kann überdies ein Enzym als Pflegestoff enthalten. Erfindungsgemäß besonders bevorzugte Enzyme sind ausgewählt aus einer Gruppe, die gebildet wird aus Proteasen, Lipasen, Transglutaminase, Oxidasen und Peroxidasen.

Obwohl jeder der genannten Pflegestoffe für sich alleine bereits ein zufrieden stellendes Resultat ergibt, sind im Rahmen der vorliegenden Erfindung auch alle Ausführungsformen umfasst, in denen das Mittel mehrere Pflegestoffe auch aus verschiedenen Gruppen enthält.

Durch Zugabe eines UV-Filters können sowohl die Mittel selbst, als auch die behandelte Haut oder die Haare vor schädlichen Einflüssen von UV-Strahlung geschützt werden. Vorzugsweise wird daher dem Mittel mindestens ein UV-Filter zugegeben. Die geeigneten UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Die erfindungsgemäß bevorzugten UV-Filter können beispielsweise ausgewählt werden aus substituierten Benzophenonen, p-Aminobenzoesäureestern, Diphenylacrylsäureestern, Zimtsäureestern, Salicylsäureestern, Benzimidazolen und o-Aminobenzoesäureestern.

Die UV-Filter sind üblicherweise in Mengen von 0,01-5 Gew.-%, bezogen auf die gesamte Anwendungszubereitung, enthalten. Mengen von 0,1-2,5 Gew.-% sind bevorzugt.

In Abhängigkeit von der Art des erfindungsgemäßen Mittels kann es erforderlich sein, dass diese weiterhin mindestens ein Tensid enthalten. Dies gilt insbesondere für Hautreinigungsmittel und Shampoos. Aber auch andere Mittel, wie beispielsweise Haarspülungen, Haarkuren und bestimmte Stylingmittel, insbesondere Stylingschäume, können Tenside enthalten.

Beispielsweise können kationische Tenside eingesetzt werden, wie sie bereits oben als geeignete Pflegestoffe beschrieben sind. Bezüglich der bevorzugten kationischen Tenside und der eingesetzten Mengen gelten obige Ausführungen entsprechend.

Neben oder statt der kationischen Tenside können die Mittel weitere Tenside oder Emulgatoren enthalten, wobei prinzipiell sowohl anionische als auch ampholytische und nichtionische Tenside und alle Arten bekannter Emulgatoren geeignet sind. Die Gruppe der ampholytischen oder auch amphoteren Tenside umfasst zwitterionische Tenside und Ampholyte. Die Tenside können bereits emulgierende Wirkung haben.

Als anionische Tenside eignen sich prinzipiell alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,

- lineare und verzweigte Fettsäuren mit 8 bis 30 C-Atomen (Seifen),
- Ethercarbonsäuren der Formel $R\text{-}O\text{-}(CH_2\text{-}CH_2O)_x\text{-}CH_2\text{-}COOH$, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremonoalkylpolyoxyethylester mit 8 bis 24 C-Atomen in der Alkylgruppe und 1 bis 6 Oxyethylgruppen,
- lineare Alkansulfonate mit 8 bis 24 C-Atomen,

- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel $R\text{-}O(CH_2\text{-}CH_2O)_x\text{-}OSO_3H$, in der R eine bevorzugt lineare Alkylgruppe mit 8 bis 30 C-Atomen und x = 0 oder 1 bis 12 ist,
- Gemische oberflächenaktiver Hydroxysulfonate,
- sulfatierte Hydroxyalkylpolyethylen- und/oder Hydroxyalkylenpropylenglykolether,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (E1-I),

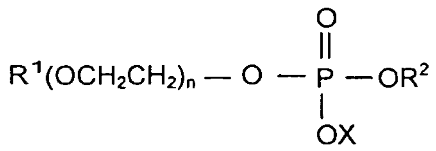

(E1-I)

in der $R^1$ bevorzugt für einen aliphatischen Kohlenwasserstoffrest mit 8 bis 30 Kohlenstoffatomen, $R^2$ für Wasserstoff, einen Rest $(CH_2CH_2O)_nR^1$ oder X, n für Zahlen von 1 bis 10 und X für Wasserstoff, ein Alkali- oder Erdalkalimetall oder $NR^3R^4R^5R^6$, mit $R^3$ bis $R^6$ unabhängig voneinander stehend für Wasserstoff oder einen C1 bis C4 - Kohlenwasserstoffrest, steht,

- sulfatierte Fettsäurealkylenglykolester der Formel (E1-II)

$$R^7CO(AlkO)_nSO_3M \qquad \text{(E1-II)}$$

in der $R^7CO$- für einen linearen oder verzweigten, aliphatischen, gesättigten und/oder ungesättigten Acylrest mit 6 bis 22 C-Atomen, Alk für $CH_2CH_2$, $CHCH_3CH_2$ und/oder $CH_2CHCH_3$, n für Zahlen von 0,5 bis 5 und M für ein Kation steht, wie sie in der DE-OS 197 36 906 beschrieben sind,

- Monoglyceridsulfate und Monoglyceridethersulfate der Formel (E1-III)

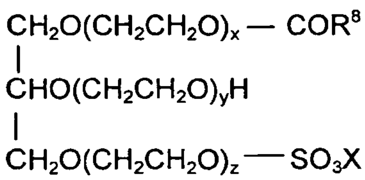

(E1-III)

in der $R^8CO$ für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (E1-III) eingesetzt, in der $R^8CO$ für einen linearen Acylrest mit 8 bis 18 Kohlenstoffatomen steht,

- Amidethercarbonsäuren,
- Kondensationsprodukte aus $C_8$ - $C_{30}$ - Fettalkoholen mit Proteinhydrolysaten und/oder Aminosäuren und deren Derivaten, welche dem Fachmann als Eiweissfettsäurekondensate bekannt sind, wie beispielsweise die Lamepon® - Typen, Gluadin® - Typen, Hostapon® KCG oder die Amisoft® - Typen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen, Monoglycerdisulfate, Alkyl- und Alkenyletherphosphate sowie Eiweissfettsäurekondensate.

Als zwitterionische Tenside werden solche oberflächenaktiven Verbindungen bezeichnet, die im Molekül mindestens eine quartäre Ammoniumgruppe und mindestens eine $-COO^{(-)}$ - oder $-SO_3^{(-)}$ - Gruppe tragen. Besonders geeignete zwitterionische Tenside sind die sogenannten Betaine wie die N-Alkyl-N,N-dimethylammonium-glycinate,

beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter Ampholyten werden solche oberflächenaktiven Verbindungen verstanden, die außer einer $C_8$ - $C_{24}$- Alkyl- oder -Acylgruppe im Molekül mindestens eine freie Aminogruppe und mindestens eine -COOH- oder -$SO_3H$-Gruppe enthalten und zur Ausbildung innerer Salze befähigt sind. Beispiele für geeignete Ampholyte sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte Ampholyte sind das N-Kokosalkylaminopropionat, das Kokosacylaminoethylaminopropionat und das $C_{12}$ - $C_{18}$ - Acylsarcosin.

Nichtionische Tenside enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise

- Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder $C_2$ - $C_6$ - Alkylrest endgruppenverschlossene Anlagerungsprodukte von 2 bis 50 Mol Ethylenoxid und/oder 1 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol® LS, Dehydol® LT (Cognis) erhältlichen Typen,
- $C_{12}$-$C_{30}$-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen® HSP (Cognis) oder Sovermol-Typen (Cognis),
- alkoxilierte Triglyceride,
- alkoxilierte Fettsäurealkylester der Formel (E4-I)

$$R^1CO\text{-}(OCH_2CHR^2)_wOR^3 \qquad \text{(E4-I)}$$

in der $R^1CO$ für einen linearen oder verzweigten, gesättigten und/oder ungesättigten Acylrest mit 6 bis 22 Kohlenstoffatomen, $R^2$ für Wasserstoff oder Methyl, $R^3$ für lineare oder verzweigte Alkylreste mit 1 bis 4 Kohlenstoffatomen und w für Zahlen von 1 bis 20 steht,

- Aminoxide,
- Hydroxymischether, wie sie beispielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

$$R^4O\text{-}[G]_p \qquad \text{(E4-II)}$$

in der $R^4$ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Die Alkyl- und Alkenyloligoglykoside können sich von Aldosen bzw. Ketosen mit 5 oder 6 Kohlenstoffatomen, vorzugsweise von Glucose, ableiten. Die bevorzugten Alkyl- und/oder Alkenyloligoglykoside sind somit Alkyl- und/oder Alkenyloligoglucoside. Die Indexzahl p in der allgemeinen Formel (E4-II) gibt den Oligomerisierungsgrad (DP), d. h. die Verteilung von Mono- und Oligoglykosiden an und steht für eine Zahl zwischen 1 und 10. Während p im einzelnen Molekül stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest $R^4$ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der

Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge $C_8$-$C_{10}$ (DP = 1 bis 3), die als Vorlauf bei der destillativen Auftrennung von technischem $C_8$-$C_{18}$-Kokosfettalkohol anfallen und mit einem Anteil von weniger als 6 Gew.-% $C_{12}$-Alkohol verunreinigt sein können sowie Alkyloligoglucoside auf Basis technischer $C_{9/11}$-Oxoalkohole (DP = 1 bis 3). Der Alkyl- bzw. Alkenylrest $R^{15}$ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem $C_{12/14}$-Kokosalkohol mit einem DP von 1 bis 3.

- Zuckertenside vom Typ der Fettsäure-N-alkylpolyhydroxyalkylamide, ein nichtionisches Tensid der Formel (E4-III),

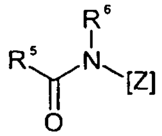

(E4-III)

in der $R^5CO$ für einen aliphatischen Acylrest mit 6 bis 22 Kohlenstoffatomen, $R^6$ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht. Bei den Fettsäure-N-alkylpolyhydroxyalkylamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können. Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher Fettsäure-N-alkylglucamide dar, wie sie durch die Formel (E4-IV) wiedergegeben werden:

$$R^7CO\text{-}NR^8\text{-}CH_2\text{-}(CHOH)_4CH_2OH \qquad (E4\text{-}IV)$$

Vorzugsweise werden als Fettsäure-N-alkylpolyhydroxyalkylamide Glucamide der Formel (E4-IV) eingesetzt, in der $R^8$ für Wasserstoff oder eine Alkylgruppe steht und $R^7CO$ für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. technischer Mischungen dieser Säuren steht. Besonders bevorzugt sind Fettsäure-N-alkylglucamide der Formel (E4-IV), die durch reduktive Aminierung von Glucose mit Methylamin und anschließende Acylierung mit Laurinsäure oder C12/14-Kokosfettsäure bzw. einem entsprechenden Derivat erhalten werden. Weiterhin können sich die Polyhydroxyalkylamide auch von Maltose und Palatinose ableiten.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Weiterhin können als nichtionische Tenside die Zuckertenside enthalten sein. Diese sind bevorzugt in Mengen von 0,1 bis 20 Gew.-%, bezogen auf die jeweilige gesamte Zusammensetzung, enthalten. Mengen von 0,5 bis 15 Gew.-% sind besonders bevorzugt, und ganz besonders bevorzugt sind Mengen von 0,5 bis 7,5 Gew.-%.

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch

solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder - alkoholate als Katalysatoren verwendet werden. Die Verwendung von Produkten mit eingeengter Homologenverteilung kann bevorzugt sein.

Die weiteren Tenside werden in der Regel in Mengen von 0,1 bis 45 Gew.-%, bevorzugt 0,5 bis 30 Gew.-% und ganz besonders bevorzugt von 0,5 bis 25 Gew.-%, bezogen auf die jeweilige gesamte Zusammensetzung, eingesetzt. Dabei hängt die eingesetzte Menge wesentlich davon ab, welchen Zweck das erfindungsgemäße Mittel erfüllt. Handelt es sich um ein Shampoo oder ein anderes reinigendes Mittel, sind auch Tensidmengen über 45 Gew.-% üblich.

Die Mittel können weiterhin mindestens einen Emulgator enthalten. Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Die Auswahl dieser emulgierenden Tenside oder Emulgatoren richtet sich dabei nach den zu dispergierenden Stoffen und der jeweiligen äußeren Phase sowie der Feinteiligkeit der Emulsion.

Die Mittel können neben den genannten Komponenten weiterhin alle für entsprechende kosmetische Mittel bekannten Wirk-, Zusatz- und Hilfsstoffe enthalten.

Weitere Wirk-, Hilfs- und Zusatzstoffe, sind beispielsweise

- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit, vollsynthetische Hydrokolloide wie z.B. Polyvinylalkohol, und gegebenenfalls vernetzte Polyacrylate, Silikate (z.B. Laponite® XLG), gelbildende Verdicker (z.B. Structure® 2001, Synthylen® 2000),
- Strukturanten wie Maleinsäure und Milchsäure,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Substanzen zur Einstellung des pH-Wertes, wie beispielsweise übliche Säuren, insbesondere Genusssäuren, und Basen,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Konservierungsmittel,
- Stabilisierungsmittel für Wasserstoffperoxid und andere Oxidationsmittel,
- Treibmittel wie Propan-Butan-Gemische, $N_2O$, Dimethylether, $CO_2$ und Luft,
- Antioxidantien.

Bezüglich weiterer fakultativer Komponenten sowie der eingesetzten Mengen dieser Komponenten wird ausdrücklich auf die dem Fachmann bekannten einschlägigen Handbücher verwiesen.

Die Formulierung der erfindungsgemäßen Mittel kann in allen für kosmetische Mittel üblichen Formen erfolgen, beispielsweise in Form von Lösungen, die als Gesichts- oder Haarwasser oder Pump- oder Aerosolspray auf die Haut oder das Haar aufgebracht werden können, in Form von Cremes, Emulsionen, Wachsen, Gelen oder auch tensidhaltigen schäumenden Lösungen oder anderen Zubereitungen, die für die Anwendung auf der Haut oder dem Haar geeignet sind. Vorzugsweise handelt es sich jedoch bei den erfindungsgemäßen Mitteln um Mittel zur temporären Verformung keratinischer Fasern, d.h. um Stylingmittel. Bevorzugte Stylingmittel sind Stylinggele, Pumphaarsprays, Aerosolhaarspray, Pumphaarschäume und Aerosolhaarschäume.

Die Formulierung der erfindungsgemäßen Mittel kann in allen für kosmetische Mittel üblichen Formen erfolgen, beispielsweise in Form von Lösungen, die als Gesichts- oder Haarwasser oder Pump- oder Aerosolspray auf die Haut oder das Haar aufgebracht werden können, in Form von Cremes, Emulsionen, Wachsen, Gelen oder auch tensidhaltigen schäumenden Lösungen oder anderen Zubereitungen, die für die Anwendung auf der Haut oder dem Haar geeignet sind. Vorzugsweise handelt es sich jedoch bei den erfindungsgemäßen Mitteln um Mittel zur temporären Verformung keratinischer Fasern, d.h. um Stylingmittel. Bevorzugte Stylingmittel sind Stylinggele, Pumphaarsprays, Aerosolhaarspray, Pumphaarschäume und Aerosolhaarschäume.

Stylinggele ist dabei im Rahmen der vorliegenden Anmeldung der Oberbegriff für klare oder trübe Produkte, Stylingwachse, Stylingcremes, Stylinglotionen, Styling-Jellys usw. Letztlich fallen unter diesen Begriff alle Mittel zum Frisieren von Haaren, die nicht Haarsprays oder Schäume sind.

Unter Haarschäumen werden dabei Zusammensetzungen verstanden, die bei der Entnahme aus einem geeigneten Behälter einen Schaum ausbilden. Es kann notwendig sein, den Mitteln Inhaltsstoffe zuzusetzen, die die Schaumbildung fördern oder einmal gebildeten Schaum stabilisieren. Insbesondere eignen sich dafür Tenside und/oder Emulgatoren, wie sie bereits oben beschrieben wurden. Vorzugsweise werden Tenside aus der Gruppe der kationischen Tenside eingesetzt. Haarcremes und Haargele enthalten in der Regel Strukturanten und/oder verdickende Polymere, die dazu dienen, den Produkten die gewünschte Konsistenz zu verleihen. Strukturanten und/oder verdickende Polymere werden typischerweise in einer Menge von 0,1 bis 10 Gew.-%, bezogen auf das gesamte Produkt, eingesetzt. Mengen von 0,5 bis 5 Gew.-%, insbesondere 0,5 bis 3 Gew.-% sind bevorzugt. Da die erfindungsgemäß eingesetzte Polymerkombination jedoch selbstverdickende Eigenschaften aufweist, ist die Zugabe weiterer Strukturanten und/oder verdickender Polymere nicht zwingend erforderlich. Vorzugsweise enthalten die erfindungsgemäßen Mittel keine weiteren Strukturanten und/oder verdickender Polymere.

Sofern es sich bei den erfindungsgemäßen Mitteln um ein Aerosolprodukt handelt, enthält dieses zwingend ein Treibmittel.

Erfindungsgemäß geeignete Treibmittel sind beispielsweise $N_2O$, Dimethylether, $CO_2$, Luft und Alkane mit 3 bis 5 Kohlenstoffatomen, wie Propan, n-Butan, iso-Butan, n-Pentan und iso-Pentan, und deren Mischungen. Bevorzugt sind Dimethylether, Propan, n-Butan, iso-Butan und deren Mischungen.

Bevorzugt werden die genannten Alkane, Mischungen der genannten Alkane oder Mischungen der genannten Alkane mit Dimethylether als einziges Treibmittel eingesetzt. Die Erfindung umfasst aber ausdrücklich auch die Mitverwendung von Treibmitteln vom Typ der Fluorchlorkohlenwasserstoffe, insbesondere aber der Fluorkohlenwasserstoffe.

Über das Mengenverhältnis von Treibmittel zu den übrigen Bestandteilen der Zubereitungen lassen sich bei gegebener Sprühvorrichtung die Größen der Aerosoltröpfchen bzw. der Schaumblasen und die jeweilige Größenverteilung einstellen.

Die Menge an eingesetztem Treibmittel variiert in Abhängigkeit von der konkreten Zusammensetzung des Mittels, der verwendeten Verpackung und der gewünschten Produktart, etwa Haarspray oder Haarschaum. Bei Verwendung herkömmlicher Sprühvorrichtungen enthalten Aerosolschaumprodukte das Treibmittel bevorzugt in Mengen von 1 bis 35 Gew.-%, bezogen auf das gesamte Produkt. Mengen von 2 bis 30 Gew.-%, insbesondere von 3 bis 15 Gew.-% sind besonders bevorzugt. Aerosolsprays enthalten generell größere Mengen an Treibmittel. Bevorzugt wird das Treibmittel in diesem Fall in einer Menge von 30 bis 98 Gew.-%, bezogen auf das gesamte Produkt, eingesetzt. Mengen von 40 bis 95 Gew.-%, insbesondere von 50 bis 95 Gew.-% sind besonders bevorzugt.

Die Aerosolprodukte lassen sich in üblicher Art und Weise herstellen. In der Regel werden alle Bestandteile des jeweiligen Mittels mit Ausnahme des Treibmittels in einen geeigneten druckfesten Behälter eingefüllt. Dieser wird daraufhin mit einem Ventil verschlossen. Über herkömmliche Techniken wird schließlich die gewünschte Menge Treibmittel eingefüllt.

Ein zweiter Gegenstand der Erfindung ist daher ein Verfahren, bei dem das erfindungsgemäße, kosmetisches Mittel auf das Haar als Pumphaarspray, Aerosolhaarspray, Pumphaarschaum, Aerosolhaarschaum oder Stylinggel aufgetragen wird und gegebenenfalls mit den Handflächen und/oder den Fingern in das Haar eingearbeitet wird.

Die gewünschte Verformung der Haare kann dabei mit den Finger oder Händen sowie mit geeigneten, herkömmlichen Hilfsmitteln wie beispielsweise Kamm oder Bürste erfolgen.

Ein dritter Gegenstand der Erfindung ist die Verwendung der erfindungsgemäßen Mittel zur temporären Verformung keratinischer Fasern.

Die erfindungsgemäßen Mittel und Produkte, die diese Mittel enthalten, zeichnen sich insbesondere dadurch aus, dass sie behandeltem Haar einen sehr starken und feuchtebeständigen Frisurenhalt verleihen.

Ein weiterer Gegenstand der vorliegenden Erfindung ist ein Verfahren, bei dem ein erfindungsgemäßes kosmetisches Mittel auf das Haar als Pumphaarspray, Aerosolhaarspray, Pumphaarschaum, Aerosolhaarschaum oder Stylinggel aufgetragen wird und gegebenenfalls mit den Handflächen und/oder den Fingern in das Haar eingearbeitet wird.

Für das erfindungsgemäße Verfahren gilt mutatis mutandis das zu den erfindungsgemäßen Mitteln Gesagte.

Ein weiterer Gegenstand der vorliegenden Erfindung ist die Verwendung von Polymermischungen, enthaltend,

bezogen auf ds Gewicht des anwendungsbereiten Mittels

a) 0,25 bis 5,0 Gew.-% mindestens eines Copolymer A, gebildet aus

- mindestens einem Monomer A1 ausgewählt aus Acrylsäure und/oder Methacrylsäure, und
- mindestens einem Monomer A2 ausgewählt aus Acrylamid und/oder Methacrylamid und
- Acryloyldimethyltaurat

b) und 0,25 bis 5,0 Gew.-% mindestens eines von Copolymer A unterschiedlichen kationischen Polymers B, ausgewählt aus

b2) Copolymeren von Vinylpyrrolidon mit Dimethylaminoethylmethacrylat,

dadurch gekennzeichnet, dass das Gewichtsverhältnis von Polymer(en) A zu Polymer(en) B 4:1 bis 1:4 beträgt, zur Verbesserung des Frisurenhaltes, insbesondere bei erhöhter Luftfeuchtigkeit. Auch für die erfindungsgemäße Verwendung gilt mutatis mutandis das zu den erfindungsgemäßen Mitteln Gesagte.

Der Halt der Verformung, auch als Frisurenhalt bezeichnet, sowie Flexibilität, Elastizität und Plastizität werden dabei im Sinne der vorliegenden Erfindung nach der Omega-Loop Methode bestimmt.

Dazu wird eine trockene Haarsträhne (Euro-Naturhaar der Firma Kerling, Klebetresse dicht, einseitig geklebt, Gesamtlänge 150 mm, freie Länge 130 mm, Breite 10 mm, Gewicht 0,9 ± 0,1 g) für 30 Sekunden bis zum unteren Rand der Abklebung in die zu untersuchende Polymerlösung getaucht. Anschließend wird die überschüssige Lösung zwischen Daumen und Zeigefinger abgestrichen, so dass 0,5 ± 0,02 g der Lösung auf dem Haar verbleiben. Die mit der zu untersuchenden Lösung gesättigte Haarsträhne wird um einen Teflon-Zylinder mit einem Durchmesser von 36 mm gewickelt und die überstehenden Enden werden mit einem Clip fixiert. Die präparierten Strähnen werden anschließend über Nacht bei 25°C und 50% relativer Luftfeuchte oder bei 25°C und 75% relativer Luftfeuchte im Klimaschrank getrocknet und konditioniert.

Die konditionierte Strähne wird vorsichtig von dem Teflon-Zylinder entfernt. Der entstandene Ω-Loop, eine ringförmige Struktur des in seiner Form durch den ausgebildeten Polymerfilm stabilisierten Haars, wird in den an der Messdose befestigten Greifer eingespannt und bis dicht über die Bodenplatte eines Universalprüfgeräts AMETEK LF Plus der Firma AMETEK Precision Instuments Europe GmbH, Produktgruppe Lloyd abgesenkt. Die gesamte Messung erfolgt im Klimaschrank unter konstanten klimatischen Bedingungen bei 25°C und 50% relativer Luftfeuchte oder bei 25°C und 75% relativer Luftfeuchte.

Um standardisierte Ausgangsbedingungen zu schaffen, startet die Messung mit dem Anfahren einer Vorlast von 0,07 N mit einer Geschwindigkeit von 30 mm $min^{-1}$. Anschließend wird der Ω-Loop mit einer Geschwindigkeit von 60 mm $min^{-1}$ um 8 mm gestaucht, wobei die dazu nötige Kraft gemessen wird. Nachdem die charakteristische Kraft $F_1$ bei der maximalen Deformation von 8 mm aufgezeichnet wurde, wird die Strähne mit 60 mm $min^{-1}$ soweit entlastet, dass sie 10 mm von der Bodenplatte abhebt. Von hier aus beginnt der nächste Zyklus, indem erneut die Vorlast von 0,07 N angefahren und die Strähne anschließend um 8 mm gestaucht wird, hierbei gelten die gleichen Geschwindigkeiten wie oben beschrieben. Die Messung eines Ω-Loops umfasst insgesamt 10 Zyklen.

Mit dieser Messmethode lassen sich vier charakteristische Parameter zur Beschreibung der mechanischen Eigenschaften von filmbildenden Polymeren bestimmen. Halt, Flexibilität, Plastizität und Elastizität lassen sich nach folgenden Formeln aus den gemessenen Kräften berechnen:

$$Halt = F_1\ [N]$$

($F_1$ entspricht der Maximalkraft der Messung)

$$Flexibilität = \frac{F_{10}}{F_1}$$

(gibt das Verhältnis der Maximalkräfte des zehnten zum ersten Zyklus an)

$$Plastizität = \frac{2 \cdot H_1 - H_{10}}{H_1}$$

(mit $H_1$ = 9 mm und $H_{10}$ = 9 mm + dauerhafte plastische Verformung der Strähne)

$$Elastizität = \frac{\dfrac{F_{10}(2mm) - F_{10}(1{,}5mm)}{0{,}5}}{\dfrac{F_1(2mm) - F_1(1{,}5mm)}{0{,}5}} = \frac{E_{10}}{E_1}$$

(zur Berechnung der Elastizität werden aus dem ersten und zehnten Zyklus jeweils die Kräfte zur Verformung um 1,5 mm und 2 mm erfasst und miteinander ins Verhältnis gesetzt).

**Patentansprüche**

**1.** Kosmetisches Mittel, enthaltend in einem kosmetisch akzeptablen Träger bezogen auf das Gewicht des anwendungsbereiten Mittels

a) 0,25 bis 5,0 Gew.-% mindestens eines Copolymers A, gebildet aus

- mindestens einem Monomer A1 ausgewählt aus Acrylsäure und/oder Methacrylsäure, und
- mindestens einem Monomer A2 ausgewählt aus Acrylamid und/oder Methacrylamid und
- mindestens einem Monomer A3 ausgewählt aus Acryloyldimethyltaurat

b) und 0,25 bis 5,0 Gew.-% mindestens eines von Copolymer A unterschiedlichen kationischen Polymers B, ausgewählt aus

b2) Copolymeren von Vinylpyrrolidon mit Dimethylaminoethylmethacrylat,

**dadurch gekennzeichnet, dass** das Gewichtsverhältnis von Polymer(en) A zu Polymer(en) B 4:1 bis 1:4 beträgt.

**2.** Mittel nach Anspruch 1, **dadurch gekennzeichnet, dass** es als Copolymer A ein Copolymer A1 enthält, welches als Monomer A1 Acrylsäure umfasst.

**3.** Mittel nach einem der Ansprüche 1 oder 2, **dadurch gekennzeichnet, dass** es als Copolymer A ein Copolymer A1 enthält, welches als Monomer A2 Acrylamid umfasst.

**4.** Mittel nach einem der Ansprüche 1 bis 3, **dadurch gekennzeichnet, dass** die Gesamtmenge an Copolymeren A, bezogen auf das Gewicht des anwendungsbereiten Mittels, 0,25 bis 3 Gew.-%, beträgt.

**5.** Mittel nach einem der Ansprüche 1 bis 4, **dadurch gekennzeichnet, dass** es als kationisches Polymer b2 Copolymere von Vinylpyrrolidon mit Dimethylaminoelhylmethacrylat enthält, die 40 bis 95 Mol.-%, vorzugsweise 42,5 bis 90 Mol.-% weiter bevorzugt 45 bis 85 Mol.-% und insbesondere 50 bis 80 Mol.-% Vinylpyrrolidon enthalten.

**6.** Mittel nach einem der Ansprüche 1 bis 5, **dadurch gekennzeichnet, dass** es, bezogen auf das Gewicht des anwendungsbereiten Mittels. 0,25 bis 3 Gew.-% Copolymer(e) b2 enthält.

**7.** Mittel nach einem der Ansprüche 1 bis 6, **dadurch gekennzeichnet, dass** der Gesamt-Polymergehall der Mittel 1 bis 15 Gew.-%, vorzugsweise 2,5 bis 12,5 Gew.-%, weiter bevorzugt 4 bis 10 Gew.-% und insbesondere 5 bis 8 Gew-% beträgt.

**8.** Verwendung von Polymermischungen, enthaltend, bezogen auf das Gewicht des anwendungsbereiten Mittels

a) 0,25 bis 5,0 Gew.-% mindestens eines Copolymers A, gebildet aus

- mindestens einem Monomer A1 ausgewählt aus Acrylsäure und/oder Methacrylsäure, und
- mindestens einem Monomer A2 ausgewählt aus Acrylamid und/oder Methacrylamid und
- mindestens einem Monomer A3 ausgewählt aus Acryloyldimethyltaurat

b) und 0,25 bis 5,0 Gew-% mindestens eines von Copolymer A unterschiedlichen kationisches Polymers B, ausgewählt aus b2) Copolymeren von Vinylpyrrolidon mit Dimethylaminoethylmethacrylat.

**dadurch gekennzeichnet, dass** das Gewichtsverhältnis von Polymer(en) A zu Polymer(en) B 4:1 bis 1:4 beträgt, zur Verbesserung des Frisurenhaltes, insbesondere bei erhöhter Luftfeuchtigkeit.

## Claims

**1.** A cosmetic agent, containing in a cosmetically acceptable carrier, with respect to the weight of the agent ready for use,

a) 0.25 to 5.0 wt% of at least one copolymer A, formed of

- at least one monomer A1 selected from acrylic acid and/or methacrylic acid, and
- at least one monomer A2 selected from acrylamide and/or methacrylamide, and
- at least one monomer A3 selected from acryloyldimethyltaurate

b) and 0.25 to 5.0 wt% of at least one cationic polymer B different from copolymer A, selected from

b2) copolymers of vinylpyrrolidone with dimethylaminoethyl methacrylat, **characterized in that** the weight ratio of polymer(s) A to polymer(s) B is 4:1 to 1:4.

**2.** The agent according to claim 1, **characterized in that** said agent contains, as copolymer A, a copolymer A1 that comprises acrylic acid as monomer A1.

**3.** The agent according to claim 1 or 2, **characterized in that** said agent contains, as copolymer A, a copolymer A1 that comprises acrylamide as monomer A2.

**4.** The agent according to one of claims 1 to 3, **characterized in that** the total amount of copolymer A, with respect to the weight of the agent ready for use, is 0.25 to 3 wt%.

**5.** The agent according to one of claims 1 to 4, **characterized in that** said agent contains, as cationic polymer b2, copolymers of vinylpyrrolidone with dimethylaminoethyl methacrylate that contain 40 to 95 mol%, preferably 42.5 to 90 mol%, more preferably 45 to 85 mol%, and particularly 50 to 80 mol%, of vinylpyrrolidone.

**6.** The agent according to one of claims 1 to 5, **characterized in that** said agent contains, with respect to the weight of the agent ready for use, 0.25 to 3 wt% of copolymer(s) b2.

**7.** The agent according to one of claims 1 to 6, **characterized in that** the total polymer content of the agents is 1 to 15 wt%, preferably 2.5 to 12.5 wt%, more preferably 4 to 10 wt%, and particularly 5 to 8 wt%.

**8.** The use of polymer mixtures containing, with respect to the weight of the agent ready for use,

a) 0.25 to 5.0 wt% of at least one copolymer A, formed of

- at least one monomer A1 selected from acrylic acid and/or methacrylic acid, and
- at least one monomer A2 selected from acrylamide and/or methacrylamide, and
- at least one monomer A3 selected from acryloyldimethyltaurate

b) and 0.25 to 5.0 wt% of at least one cationic polymer B different from copolymer A, selected from b2) copolymers of vinylpyrrolidone with dimethylaminoethyl methacrylat,

**characterized in that** the weight ratio of polymer(s) A to polymer(s) B is 4:1 to 1:4, to improve hairstyle hold, particularly at elevated humidity.

## Revendications

1. Composition cosmétique contenant dans un support cosmétiquement acceptable, sur la base du poids de la composition prête à l'emploi,

    a) de 0,25à 5,0% en poids d'au moins un copolymère A formé

    - d'au moins un monomère A1 choisi parmi l'acide acrylique et/ou l'acide méthacrylique et
    - au moins un monomère A2 choisi parmi l'acrylamide et/ou le méthacrylamide et
    - au moins un monomère A3 choisi parmi l'acryloyldiméthyllaurate,

    b) et de 0,25 à 5,0% en poids d'au moins un polymère cationique B, différent du copolymère A, choisi parmi

    b2) les copolymères de la vinylpyrrolidone avec le diméthylaminoéthylméthacrylate,

    **caractérisée en ce que** le rapport en poids du ou des polymères A sur le ou les polymères B va de 4:1 à 1:4.

2. Composition selon la revendication 1, **caractérisée en ce qu'**elle contient comme copolymère A un copolymère A1 qui contient comme monomère A1 l'acide acrylique.

3. Composition selon l'une des revendications 1 ou 2, **caractérisée en ce qu'**elle contient comme copolymère A un copolymère A1 qui contient comme monomère A2 l'acrylamide.

4. Composition selon l'une des revendications 1 à 3, **caractérisée en ce que** la quantité totale de copolymère A, sur la base du poids de la composition prête à l'emploi, est de 0,25 à 3% en poids.

5. Composition selon l'une des revendications 1 à 4, **caractérisée en ce qu'**elle contient comme polymère cationique b2 des copolymères de la vinylpyrrolidone avec le diméthylaminoéthylméthacrylate qui contiennent de 40 à 95% en mole, de préférence de 42,5 à 90% en mole, plus préférablement de 45 à 85% en mole et en particulier de 50 à 80% en mole de vinylpyrrolidone.

6. Composition selon l'une des revendications 1 à 5, **caractérisée en ce qu'**elle contient, sur la base du poids de la composition prête à l'emploi, de 0,25 à 3% en poids de copolymère(s) b2.

7. Composition selon l'une des revendications 1 à 6, **caractérisée en ce que** la teneur totale en polymères de la composition est de 1 à 15% en poids, de préférence de 2,5 à 12,5% en poids, plus préférablement de 4 à 10% en poids et, en particulier de 5 à 8% en poids.

8. Utilisation de mélanges de polymères contenant, sur la base du poids de la composition prête à l'emploi,

    a) de 0,25à 5,0% en poids d'au moins un copolymère A formé

    - d'au moins un monomère A1 choisi parmi l'acide acrylique et/ou l'acide méthacrylique et
    - au moins un monomère A2 choisi parmi l'acrylamide et/ou le méthacrylamide et
    - au moins un monomère A3 choisi parmi l'acryloyldiméthyllaurate,

    b) et de 0,25 à 5,0% en poids d'au moins un polymère cationique B, différent du copolymère A, choisi parmi b2) les copolymères de la vinylpyrrolidone avec le diméthylaminoéthylméthacrylate,
    **caractérisée en ce que** le rapport en poids du ou des polymères A sur le ou les polymères B va de 4:1 à 1:4, pour améliorer le maintien de la coiffure, en particulier lorsque l'humidité de l'air est élevée.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- DE PS4413686 C
- DE 19756454 A
- DE OS19736906 A
- DE OS19738866 A

### In der Beschreibung aufgeführte Nicht-Patentliteratur

- International Cosmetic Ingredient Dictionary and Handbook. The Cosmetic, Toiletry, and Fragrance Association, 1997